# EUROPEAN PATENT APPLICATION

(11) **EP 0 798 397 A2**
(43) Date of publication of application: **01.10.1997**
(21) Application number: 97302091.0
(22) Date of filing: 26.03.1997
(51) Int. Cl.: C23C 8/38, A61B 17/06

(54) **Process for cleaning surgical needles**

(30) Priority: 27.03.1996 US 625437
(71) Applicant: ETHICON, INC., Somerville, N J 08876 (US)
(72) Inventor: Vetrecin, Robert, Stewartsville, NJ 08886 (US); Hersey, Bruce, San Angelo, TX 76901 (US)
(74) Representative: Fisher, Adrian John

(57) **Abstract**

A process for cleaning surfaces of a metal alloy surgical needle or a metal alloy surgical instrument. The process consists of exposing the surfaces of a metal alloy surgical needle or surgical instrument to a gaseous plasma for a sufficient amount of time to effectively clean the surfaces of the needle or instrument.

## Description

### Technical Field

The field of art to which this invention pertains is surgical needles. In particular, a method of treating surgical needles and surgical needles produced by such a method.

### Background of the Invention

Surgical needles are typically manufactured from wire, in particular, various conventional grades of stainless steel and equivalent alloys which have corrosion resistance and desired mechanical properties. Typical alloys include Types 420, 420F and 455 stainless steels and nickel titanium martensitic stainless steel alloys such as those disclosed in U.S. Patent No. 5,000,912 which is incorporated by reference. The wire is typically received from the manufacturer wound onto a spool. In a typical surgical needle manufacturing process, the wire is removed from the spool, straightened, and then cut into needle blanks. The needle blanks are then subjected to a series of manufacturing steps wherein piercing points are ground or formed, suture mounting sections are drilled or formed, and the needle is shaped to have the final geometry which may include cutting edges, tapers, curved sections, etc. Processes for manufacturing surgical needles are disclosed in commonly assigned, co-pending U.S Patent Application Serial Nos. 08/405,554 and 08/429,446 which are incorporated by reference.

It is known that the wire which is used to manufacture the needles has surface contaminants when it is received from the mill. In addition, additional surface contaminants may be picked up during the manufacturing process. These contaminants, which are well known in this art, may include drawing lubricants, processing lubricants, greases, oils, carbon, other hydrocarbon materials and the like.

Although the existing cleaning processes produce needles having adequately cleaned surfaces, there are certain disadvantages associated with the use of these processes. The processes may require the use of chemical baths, either aqueous or organic, generating both chemical fumes and hazardous waste. The chemical baths have a limited lifetime and have to be disposed of at considerable expense. In addition, the use of the types of chemicals required for the cleaning baths has with it attendant safety hazards which must be constantly monitored. A further disadvantage of conventional cleaning processes is that residues from the cleaning baths or contaminants from the manufacturing process may be left on the needles or instruments, even after repeated conventional cleaning cycles.

Accordingly there is a need in this art for a novel method of cleaning the surfaces of metal alloy surgical needles and surgical instruments without the use of chemical baths.

### DISCLOSURE OF THE INVENTION

It is an object of the present invention to provide a process for cleaning the surfaces of metal alloy surgical needles and surgical instruments without the use of chemical baths.

It is a further object of the present invention to provide a process for cleaning the surfaces of surgical needles or instruments which eliminates residual contaminants.

Therefore, a process for cleaning a metal alloy surgical needle or surgical instrument is disclosed. The metal surgical instrument or surgical needle has an exterior surface, and optionally an interior surface. The surfaces of the metal surgical needle or surgical instrument are exposed to a gaseous plasma for a sufficient period of time at a sufficient temperature to effectively clean contaminants from the surfaces of the needle or instrument.

Yet another aspect of the present invention is a surgical needle or instrument cleaned by the above-described process.

The novel method of cleaning metal alloy surgical needles or surgical instruments of the present invention has many advantages. Environmental hazards associated with the use of chemical baths a are eliminated since these chemical baths are not needed. In addition, the gaseous plasma which is used to clean the exterior surface of the surgical instruments and the surgical needles may be recaptured and recycled after each process. In addition, the present plasma process produces needles which have surfaces which may be cleaner than needles cleaned by conventional processes.

The foregoing and other features and advantages of the present invention will become more apparent from the following description and accompanying drawings.

### BRIEF DESCRIPTION OF THE DRAWINGS

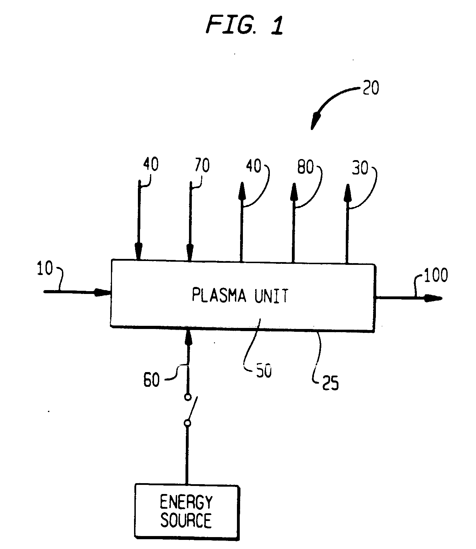

FIG. 1 illustrates a flow diagram for a cleaning process of the present invention.

### BEST MODE FOR CARRYING OUT THE INVENTION

The gases which can be used for the plasmas of the present invention include oxygen, helium, carbon tetrafluoride, nitrogen, argon and the like and mixtures thereof and equivalents thereof. It is particularly preferred to use mixtures of gases such as oxygen, helium, and carbon tetrafluoride. When using a mixture of oxygen, helium or argon, and carbon tetrafluoride, sufficient amounts of each component will be utilized to provide an effective plasma mixture. Typically, about 50% to about 99% of oxygen will be used in such a mixture, more typically about 60% to about 99%, and preferably about 70% to about 99%. The typical amounts of helium or argon which will be mused in such a mixture will typically be about 1% to about 50%, more typically about 10% to about 40%, and preferably about 10% to about 30%. Although not preferred, it is possible to utilize a mixture of helium and argon. The amounts of carbon tetrafluoride which will be utilized in a preferred mixture will typically be about 1% to about 40%, more typically about 15% to about 35%, and preferably about 10% to about 30%. The percentages used herein are percentages by weight. A particularly preferred cleaning mixture will utilize about 10% of Helium or Argon, about 70% of Oxygen, and about 20% of carbon tetrachloride.

A conventional plasma treatment process unit is typically used in the processes of the present invention. The plasma unit will typically have a volumetric chamber which is capable of withstanding both pressure and vacuum. Mounted in the chamber will be at least one electrode. A preferred unit is a Gasonics' plasma unit manufactured by Gasonics/IPL, San Jose, California, however, any conventional or equivalent gas California, however, any convertional or equivalent gas plasma unit may be utilized as well as any primary or secondary discharge unit. A gaseous mixture or gas is typically brought up to a plasma state in these units by exposing the gas to sufficient electromagnetic energy such as radio frequency electromagnetic waves, microwaves, etc., to effectively induce a plasma state. However, other means of exciting the gas into a plasma state may be utilized including direct current, laser energy, equivalents thereof and the like. If desired, the needles or instruments may exposed to a conventional plasma torch.

Sufficient electromagnetic energy will be applied to the gas to effectively produce a plasma condition. Typically the amount of energy utilized will be-about 250W (0.12 watts/M²) to about 2500W (1.2 watts/M²), more typically to about 300W (.014 watts/M²) to about 1000W (0.46 watts/M²), and preferably about 400W (0.18 watts/M²) to about 900W (0.42 watts/M²). Of course those skilled in the art will appreciate that the amount of energy utilized will vary in accordance with the process parameters including gas flow, gas type, electrode area, and vacuum, etc., as well as the type, size, condition and configuration of the plasma unit.

The gas will flow into the plasma treatment process unit at a sufficient volumetric flow rate to effectively produce a plasma. The volume of the chamber of the unit will be sufficient to effectively contain the needles or instruments being treated. These parameters will vary in accordance with the particular parameters of the process and are readily determined by those skilled in the art.

A typical flow diagram for a cleaning process of the present invention is illustrated in FIG. 1, although those skilled in the art will appreciate that various steps may be eliminated or added to the processes of the present invention. As illustrated in FIG. 1, the initial step of such a process is to load needles or surgical instruments 10 into a chamber 25 of a plasma treatment unit 20. Next, a sufficient vacuum 30 is pulled on the chamber 25 to effectively evacuate the chamber of air and produce a sufficient vacuum to introduce the plasma. Typically, the vacuum may be about 0.05 to about 1 Torr, more typically about 0.25 to about 0.75 Torr, and preferably about 0.3 to about 0.5 Torr, however this will vary with the type and configuration of plasma unit utilized. Then, the chamber 25 is filled with a gas or gas mixture 40 of choice so that there is a sufficient amount of gas 40 present in the chamber 25 to effectively form the gas plasma 50. Typically the flow rate of gas 40 utilized may be about 50 to about 500 cc/min, more typically about 100, to about 500 cc/min, and preferably about 200 to about 500 cc/min, however, this flow rate may vary depending upon the type and configuration of the plasma unit utilized. Next, the power is switched on energizing the energy source 60 within the unit 20 and thereby forming a plasma 50, and the needles or surgical instruments 10 are exposed to the gas plasma 50 for a sufficient period of time to effectively clean the surfaces of the needles or surgical instruments 10. Typically the plasma cycle time may be about 10 to about 60 minutes, more typically about 20 to about 40 minutes, and preferably about 30 to about 45 minutes, however, this will vary depending upon the process cycle, process parameters, and plasma unit type and configuration, utilized. Next, after energy 60 has been turned off, the gas 40 is removed from chamber 25 through vent 80 and the chamber 25 is back-filled with an inert gas 70, such as nitrogen, and maintained at a sufficient pressure for a sufficient amount of time to effectively cool down the needles or instruments 10. For example, the needles may be held in the cool down phase for about 3 to about 10 minutes, more typically about 3 to about 7 minutes, and preferably about 3 to about 5 minutes. The inert gas pressure may be, for example, about 0.05 Torr to about 1.0 Torr. Finally, the cleaned needles or instruments 100 are removed from the chamber 25 of the gas plasma unit 20. The gas 40 removed through vent 80 may be recycled for use in the process.

The surgical needles which can be cleaned using the process of the present invention include any conventional surgical needle having a piercing point, sharp or blunt, and a suture mounting end. The suture mounting end may have a channel or a blind drilled hole for receiving sutures. The surgical instruments which may be cleaned using the process of the present invention include conventional instruments and parts thereof such as needle graspers, scissors, forceps, scalpels, catheters, cutting instruments, clamps, saws, and the like. The term surgical instrument as used herein is defined to include a metallic part of a surgical instrument. the needles and instruments will have exterior surfaces and may have interior surfaces.

The following example is illustrative of the principles and practice the present invention.

### Example

Approximately 30,000 surgical needles made from 420 stainless steel metal alloy were placed into the chamber of a Gasonics® Plasma Unit. The chamber had a volume of about 4 ft³. The door to the unit was sealed and the chamber was evacuated under a vacuum of about 0.15 Torr for about one minute to purge volatiles and contaminants. The chamber was next back-filled with a mixture of 50 cc/minute of oxygen and 50 cc/minute of Helium to a pressure of about 0.3 to about 0.5 Torr. The gaseous mixture was maintained in the chamber prior to switching power on for about 2 minutes. Next, power was switched on to the unit and the needles were exposed to the resulting gaseous plasma which was maintained at a power level of about 500 watts for amount 30 minutes. Next, the power was switched off and the gaseous mixture was evacuated from the chamber. Then, the chamber was filled with nitrogen and held at a pressure of about 1.0 Torr for approximately 3-5 minutes until the needles were cool enough to be handled. The needles were then removed from the chamber of the gas plasma unit. Upon inspection, the surfaces of the needles were cleaned.

The process of the present invention for cleaning the surfaces of metal alloy surgical needles and surgical instruments has many advantages. Surprisingly and unexpectedly, it is now possible to clean the surfaces of metal alloy surgical needles and surgical instruments in a controlled process which does not utilize chemical and which does not generate the associated fumes, emissions and hazardous waste streams. Using the plasma process of the present invention, it is possible to recycle the gaseous plasma agents. Yet another advantage of the present invention is that needles which are processed in the gas plasma processes of the present invention are not subjected to the metal removal which is a characteristic disadvantage of the chemical or electrochemical processes. Still yet another advantage of the process of the present invention is that it is significantly more economical and cost effective than the prior art conventional cleaning processes. The process of the present invention also eliminates the safety concerns associated with the conventional cleaning processes. Still yet another advantage of the present invention is that surfaces may be cleaned more effectively to remove more contaminants and eliminate residuals than the cleaning processes of the prior art to produce levels of cleanliness not previously produced.

Although this invention has Deen shown and described with respect to detailed embodiments thereof, it will be understood by those skilled in the art that various changes in form and detail thereof may be made without departing from the spirit and -scope of the claimed invention.

## Claims

1. A method of cleaning the surface of a metallic surgical needle, the method comprising:
exposing a metallic surgical needle having an exterior surface to a gaseous plasma for a sufficient period of time at a sufficient temperature to effectively clean the exterior surface of the needle.

2. A method of cleaning the surface of a metallic surgical instrument, the method comprising:
exposing a metallic surgical instrument having an exterior surface to a gaseous plasma for a sufficient period of time at a sufficient temperature to effectively clean the exterior surface of the instrument.

3. The method of claim 1 or claim 2 wherein the gaseous plasma comprises a mixture of oxygen and helium and carbon tetrafluoride.

4. The method of claim or claim 2 wherein the gaseous plasma comprises a mixture of oxygen, argon and carbon tetrafluoride.

5. The method of claim 1 or claim 2 wherein the plasma mixture comprises about 50 wt.% to about 99 wt.% of oxygen and about 1 wt.% to about 50 wt.% of helium and about 1 wt.% to about 40 wt.% of carbon tetrafluoride.

6. The method of claim 1 wherein the metallic needle comprises an alloy selected from the group consisting of T-420 stainless steel, T-420F stainless steel, T-455 stainless steel, and titanium nickel martensitic stainless steel alloy.

7. The method of claim 2 wherein the metallic surgical instrument comprises an alloy selected from the group consisting of T-420 stainless steel, T-420F stainless steel, T-455 stainless steel, and titanium nickel martensitic stainless steel alloy.

8. The method of any preceding claim wherein the gaseous plasma is excited by an energy source, wherein said energy source comprises a member selected from the group consisting of Radio Frequency, Microwave and DC Discharge.

9. The method of claim 1 or claim 2 wherein the plasma comprises oxygen.

10. The method of claim 2 wherein the instrument additionally comprises at least one interior surface, and the interior surface is also cleaned.

11. The method of claim 1 wherein the needle additionally comprises at least one interior surface, and the interior surface is also cleaned.
